Europäisches Patentamt

European Patent Office

Office européen des brevets

(19)

(11) **EP 1 214 933 A1**

(12) **EUROPEAN PATENT APPLICATION**
published in accordance with Art. 158(3) EPC

(43) Date of publication:
**19.06.2002 Bulletin 2002/25**

(21) Application number: **00963185.4**

(22) Date of filing: **11.09.2000**

(51) Int Cl.⁷: **A61K 9/107**, A61K 31/02

(86) International application number:
**PCT/RU00/00362**

(87) International publication number:
**WO 01/19341 (22.03.2001 Gazette 2001/12)**

(84) Designated Contracting States:
**AT BE CH CY DE DK ES FI FR GB GR IE IT LI LU MC NL PT SE**

(30) Priority: **10.09.1999 RU 99119464**

(71) Applicant: **Institut Molekulyarnoi Genetiki Rossiiskoi Akademii Nauk ( Img Ran ) Moscow 123182 (RU)**

(72) Inventors:
 • **BEDA, Nataliya Vladimirovna**
  **Moscow, 117312 (RU)**

 • **GORDIN, Vladimir Alexandrovich**
  **Moscow, 125503 (RU)**
 • **NEDOSPASOV, Andrei Arturovich**
  **Moscow, 117312 (RU)**
 • **RAFIKOV, Ruslan Robertovich**
  **Moscow, 117279 (RU)**
 • **RAFIKOVA, Olga Valerievna**
  **Moscow, 117279 (RU)**
 • **SUNTSOVA, Tatiyana Pavlovna**
  **Dolgoprudny, Moskovskaya obl., 141700 (RU)**

(74) Representative: **Cabinet Hirsch
 34, Rue de Bassano
 75008 Paris (FR)**

(54) **METHOD FOR MODULATING THE METABOLISM OF NITROGEN OXIDES, COMPOSITIONS THEREFOR (AND VARIANTS) AND METHOD FOR ACTING ON A PATIENT'S ORGANISM NECESSITATING THE METABOLISM OF NITROGEN OXIDES TO BE CORRECTED**

(57)    The invention relates to a process of modification of the nitrogen oxides metabolism by means of modification of the micellar catalysis parameters of NO oxidation. According to the invention, the number of phases and/or the volume ratio of the phases and/or the coefficients of distribution of NO and $O_2$ between the phases are modified. The number of phases is modified by using perfluorocarbons, haloid derivatives thereof and perfluoralkylamines with a high coefficient of distribution of NO and $O_2$, which are used as a hydrophobic phase for micellar analysis. The invention also relates to compositions used to vary the output of nitrite, nitrate, nitrosothiols and other oxidation products, whereby said compositions include emulsions of perfluororganic compounds, catalysts and inhibitors of excessive nitrosation, reducers, free radical scavengers and nitrosation targets which modify the balance of the nitrosated biogeneous compounds. The inventive methods for acting on a patient's organism include using such compositions together with variations in temperature and moisture and with traditional drugs. Independent claims in this invention also relate to the use of the known blood replacement substances containing perfluorated compounds and of the steam bath or the sauna in order to accelerate NO oxidation.

**EP 1 214 933 A1**

## Description

### Technical field

The invention relates to the field of chemistry in particular, to the regulation of NO-dependent processes in live organisms.

### Prior art

Nitric oxide (NO) is the most important inorganic metabolite of all higher animals [1-5]. It takes part in controlling diameter of blood vessels [3] and septic shock [6], in transmission and storage of information [4]; it is an initial product for the synthesis of peroxynitrite that kills pathogenic bacteria and cancer cells [7, 8] and acts as an absorber of free radicals [9, 10]. In mammals NO formation results from arginine oxidation by oxygen as affected by NO-synthases [4, 11] and NO degradation occurs in further oxidation. Metabolism disorders of nitric oxides causes a number of diseases; on the contrary, the methods that allow correcting metabolism of nitric oxides (for example a physiological NO donor, nitroglycerin that is used in heart failure) are effective for preventing and treating numerous pathological conditions. In a gas phase NO is oxidized by oxygen to $NO_2$. In diluted aqueous solutions two main competing NO oxidation routes simulating in vivo conditions are known: (1) trielectronic oxidation when complexes of transitional metals, such as Hb-$O_2$ induce fast and irreversible NO oxidation into nitrate; (2) monoelectronic oxidation when in absent transitional metals free $O_2$ oxidizes NO to nitrite or nitrosocompounds with intermediate formation of $N_2O_3$ [1, 5, 12].

$$NO + Hb^{+2}\text{-}O_2 \rightarrow NO^-_3 + Hb^{+3} \tag{1}$$

$$2\,NO + O_2 \rightarrow 2NO_2 \leftrightarrow N_2O_4 \tag{2}$$

$$NO_2 + NO \leftrightarrow N_2O_3 \tag{3}$$

$$N_2O_3 + H_2O \rightarrow 2NO^-_2 + 2H^+ \tag{4}$$

$$N_2O_3 + 2RSH \rightarrow 2RSNO + 2NO^-_2 + 2H^+ \tag{5}$$

In vivo nitrite and nitrosothiols can be again reduced into NO [5, 13-15]. Thus the rate ratio of the processes (1) and (2-5) controls the pool of NO and NO-equivalents and thereby the most important organism functions under both normal and pathological conditions [5, 10, 16].

NO donors, such as organic nitrates, inhibitors of NO-synthases, such as nitroarginine and enzymes destructing arginine (the NO precursor) such as arginases and others, are used for correcting metabolism of nitric oxides [5].

Increase in the concentration of reacting substances (a totally third order reaction (2)) achievable by different methods and elevating temperature are the known methods of accelerating chemical reactions including the reaction (2). For biochemical and medical applications this route finds use, for example by administering NO donors of nitroglycerine type or inhaling NO but this route is limited since the admissible NO and oxygen concentrations are physiologically predetermined. In many cases (for example in septic shock) acceleration of the reaction (2) is aimed at necessity of decrease in local NO concentration with simultaneous increase in the concentration of oxidation products (such as nitrosothiols that perform different physiological functions including NO transport in the form of NO equivalents).

The method of NO oxidation acceleration resulting from micellar catalysis is also known [1,2] in which a hydrophobic phase acts as a sponge concentrating reagents in a small volume. In [1] the method was realized experimentally by adding emulsions comprising hydrophobic components, such as liposomes to a homogenous aqueous solution. This lead to increase in the reaction rate observed by decrease of NO concentration, i.e. oxidation rate in a heterogeneous medium was shown to be higher than in a homogenous one while the products formed in oxidation of NO as such were not determined. At the same time in [1] it was a priori assumed that the dependence of the achieved acceleration on the hydrophobic phase is monotonic (i.e. the reaction rate is constantly growing with increase in hydrophobic phase portion). The document [2] can be considered the closest analog of the suggested technical solution.

Said publication theoretically showed that this dependence achieves maximum in relatively small proportions of the hydrophobic phase: i.e. in further increase in the hydrophobic phase portion the reaction rate drops. A formula for calculating NO oxidation process in multi-component systems is suggested. This document can be taken for a prototype. However in [2], practically useful method for accelerating monoelectronic NO oxidation reaction in a heterogeneous medium of a live organism, such as blood was not suggested. The published text of article also comprised an algebraic error in the formula. The paper [17] has analyzed the errors encountered in the previous publications [1,2].

The prior art does not teach the way of modulating metabolism of nitric oxides is known from (i.e. accelerating or slowing down NO oxidation reaction and subsequent conversions) in the initially heterogeneous system (in one of the practically important cases in blood) without changing neither the amounts of reacting substances nor temperature and pressure in the system. The methods that allow changing relation between the reaction products of mono- and tri-electronic oxidation without adding additional reagents are also unknown.

Stabilized emulsions based on fluorinated organic compounds used as blood substitutes with gas transporting function such as "Perftoran" are known [18]. A patent was granted for using such emulsions as reservoirs (depots) for nitric oxide [19]. Numerous clinical applications of blood substitutes based on stabilized emulsions of fluorinated organic compounds are also known [18-22].

The peptide Met-Glu-His-Phe-Pro-Gly-Pro being the base of pharmaceutical preparations of nootropic action is known [23]. The effect thereof on renitrosation is unknown.

While the therapeutic effects of the Russian steam bath and sauna are known but the character and mechanisms of their effect on metabolism of nitric oxides are still unknown.

**Disclosure of the invention**

The suggested technical solution on modulating metabolism of nitric oxides by varying NO oxidation rate consists in changing the reaction medium so that at least one novel phase would appear therein and/or the ratio between the volumes of the phases would change and/or the values of distribution coefficients $Q_{NO}$ and/or $Q_{O2}$ at least for one pair of phases would change so that the value of the expression

$$H = \frac{\sum\limits_{i=1}^{i=n-1} k_i Q_{NO,i}^2 Q_{O_2,i} x_i + k_n \left(1 - \sum\limits_{i=1}^{i=n-1} x_i\right)}{\left(1 + \sum\limits_{i=1}^{i=n-1} Q_{NO,i} x_i - \sum\limits_{i=1}^{i=n-1} x_i\right)^2 \left(1 + \sum\limits_{i=1}^{i=n-1} Q_{O_2,i} x_i - \sum\limits_{i=1}^{i=n-1} x_i\right)}$$

*where H is acceleration of NO oxidation reaction with oxygen in a heterogeneous n-phasic system as compared to the least hydrophobic (aqueous) phase, $k_i$ is the reaction rate constant in i phase, $Q_{Noi}$, $Q_{O2i}^-$ is a equilibrated distribution coefficient of NO and $O_2$ in i phase, $x_i$ is a portion of i phase in a total volume,*
describing a total reaction (2) acceleration trough the whole system (in all phases) would change.

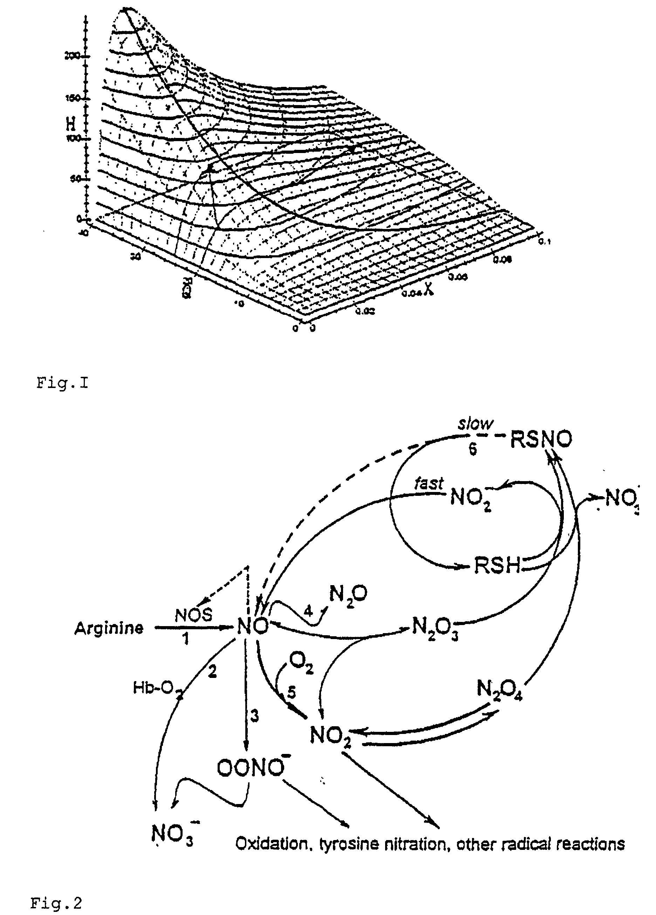

This can be achieved by change in the concentration of substances in separate phases even without introducing new components into a system. For example, in perspiration in a steam bath tissues become dehydrated that lead to an increase in the concentration of dissolved substances in aqueous phases and raises the volume portion of hydrophobic phases. The former results in rise in Q values, and the latter results in rise in x values. As a result the bot total NO oxidation rate and H value change. The result can be also achieved by introducing a more hydrophobic component (such as perfluorohydrocarbon) with a high value of $Q_{NO}$ and/or $Q_{O2}$ into the reaction medium. The dependence of H on the portion of the hydrophobic phases (x) for heterogeneous systems being really encountered in vivo, has the maximum value at relatively low values of x (several percents from a total volume); at the same time H quickly grows with increase in Q (see Fig.1). Thus as the volume of the novel phase having high Q values grows, acceleration of the reaction (2) first grows fast, achieves the maximum and then gradually drops. For real heterogeneous in vivo systems in a sufficiently large amount of the novel hydrophobic phase the reaction acceleration due to micellar catalysis becomes lower than in the initial system, i.e. the value of H decreases. Actually, the organism tissues such as blood are heterogeneous systems already before administering fluorine-comprising compounds, and therefore micellar catalysis is also efficient for them (the role of hydrophobic phases being plaid by lipids, lipoproteins and cellular membranes). Acceleration of the total (by all phases) oxidation process can be achieved only within a relatively narrow zone of concentrations of the administered fluorine comprising compound; in further increase in a volume portion thereof the total reaction rate will drop and finally it will become lower than it was before adding said compound. Thus increase in the phase volume with the maximum high Q values can both increase (the left arrow in Fig.1) and decrease (the right arrow in Fig.) the total NO oxidation rate according to the reaction (2).

The suggested group of inventions has inventive step. Actually the US patent was granted [19] claiming the use of perfluorohydrocarbones for raising NO concentration in a patient organism. The inventors supposed that NO would be diluted in the hydrophobic phase but they neglected oxidation.

A simultaneous increase in the rate of radical reactions and trielectronic NO oxidation with formation of nitrate is the above disclosed general method's peculiarity which in some cases proves to be advantageous and in a number of cases it is a drawback. The reason for this event consists in the fact that $N_2O_3$ salvation that promotes stabilization thereof in hydrophobic phases is lower than in water, in particular in the phases with a high level of fluororganic compounds. Accordingly the system equilibration of higher nitric oxides shifts towards $NO_2$ and $N_2O_4$ formation. The former promotes the course of radical reactions including nitrotyrosine formation, the latter enters reactions of electrophylic nitrosation nitrate being the second product thereof (see Fig.2).

In order to create a possibility of modulating this process also, i.e. to enhance or to weaken it depending on the necessity, several modifications are suggested in the claimed group of inventions. Possibility of controlling the time of affecting metabolism of nitric oxides is provided by using perfluorocompounds of different molecular weight and structure, said compounds controlling elimination rate thereof from the body.

According to the suggested method, in order to shift equilibration towards $N_2O_3$ renitrosation catalysts that promote the course of $N_2O_3$ reactions and other nitrosating agents (such as N-nitrosotriptophane comprised in proteins with nitrosation targets in particular water (4), thiols (5) or amines) are additionally introduced into the heterogeneous medium in which NO oxidation occurs. The necessity of simultaneous administering several catalysts and/or inhibitors can be caused by the presence of many parallel reactions since catalytic effects for each of the catalyst/reaction pairs are individual. NO oxidation rate according to the reaction (2) remains unchanged but the steady state $N_2O_3$ concentration dropping as the degradation rate thereof increases. Accordingly steady state concentrations of $NO_2$ and $N_2O_4$ being in a dynamic equilibration therewith and the rates of radical reactions and nitrate formation drop. These catalysts and inhibitors can be administered into both aqueous phase and micellars of hydrophobic phase. Intranasal administering peptide catalysts of renitrosation which rapidly getting into cerebral tissues will promote protection from damages of the specific binding sites for example can accompany Bath procedure.

Increase in the area of the interface between phases is the second way of solving this problem since the concentration of nitrosation targets in the extremely hydrophobic phase is usually low (in particular due to a low concentration of water that is one of the main nitrosation targets) and the reaction runs mainly due to reagent diffusion through the interface into aqueous phase. This variant is practically achieved by decrease in the average size of hydrophobic phase while volume portion thereof being maintained.

The third way consists in adding into the reaction mixture the targets capable of penetrating into hydrophobic phases, to nitrosate therein and to eliminate nitrous group into aqueous phase. Due to the possibility of renitrosation not only for $N_2O_3$ but also for other nitrosocompounds in particular nitrosothiols and nitrosoamines, thiols and amines can perform such transport function. However, since nitrosoamines can be cancerogenic, thiols are preferable.

The forth way is the lowering of the reaction medium temperature since NO oxidation rate by oxygen is weakly dependent on temperature and $N_2O_3$ stability rapidly falls with rise in temperature.

The fifth way of equilibration shifting towards $N_2O_3$ is increase in steady state concentration of NO reversibly interacting with $NO_2$. It can be achieved by administering NO itself e.g. by inhalation, or of precursors thereof e.g. organic nitrates, by the activity enhancement of NO-synthases, for example by increasing in concentration of substrates and cofactors thereof, or by enhancement of their expression, or by NO resynthesis acceleration from nitrite and nitrosothiols. The latter is achieved by administering reducers such as ascorbic acid or salts thereof. Complexes of transitional metals such as copper can act as catalysts. Introduction of scavengers (absorbers) of active radicals that differ from NO into the compositions penetrating into hydrophobic phase is also achievable for instance by using lipophilic tocopherol derivatives (tocopherol acetate).

The sixth way of equilibration shifting toward monoelectronic oxidation is increase in efficient nitrosation targets in the aqueous phase, the local $N_2O_3$ concentration at the interface of phases falling due to chemical reactions that results in formation of a steeper gradient and accelerating diffusion and transport of higher nitric oxides from the hydrophobic phase into the aqueous one so that the steady state $NO_2$ and $N_2O_4$ concentration decrease. In one option of this method nitrosation on amine group of the targets if followed by split off a water molecule and degrade with release of free nitrogen. Thus NO-equivalent (an NO oxidation product) is irreversibly removed from the system (unlike nitrite and nitrothiols that are capable of repeated reduction into NO - see Fig.2) that results in decrease in a total pool of NO-equivalents in the system. Biologically compatible non-toxic amides with unsubstituted amine group, urea, salts of carbamic, sulfaminic and amidophosphoric acids and hydrophobic primary amines can be used as the targets of such type for reactions in anhydrous phases.

Shifting equilibration of nitric oxides towards $NO_2$ and $N_2O_4$ (i.e. increase in the proportion of radical reactions and trielectronic oxidation) is achieved by rise in concentration of oxygen and renitrosation inhibitors, by elevation of temperature, by decrease in area of interface surface between phases, by lowering concentration of the efficient targets and by excluding hydrophobic targets capable of nitrosation in hydrophobic phases and renitrosation catalysts (denitrosilases).

Simultaneously, the compositions that allow achieving metabolism modulation of nitric oxides according to different variants of the suggested method are claimed. Patent protection being claimed for the use for a new purpose of the known stabilized emulsions of inert fluorocompounds that are used as blood substitutes with gas transport function. In addition to accelerating NO oxidation those very compositions can be used for slowing down thereof. Thus the known blood substitutes based on fluorocompounds and possessing a gas transport function can be used for both accelerating (in small relative volumes of the added hydrophobic compounds) and slowing down the process (in large volumes). The border between acceleration transitions into slowing down is determined by the equation for H acceleration value. Besides in clinical practice it is often necessary to remove the excess of NO and oxidation products thereof just from the aqueous phases (e.g. in septic shock when NO excess generated by macrophages results in a dangerous fall in blood pressure). Thus depending on the requirements in an individual case of using the invention, emulsions based on fluorinated organic compounds acts as a modulator i.e. either accelerator (H rises) or inhibitor (H subsides) of the total process or for transferring it into artificial hydrophobic phase (probably also if H is constant).

For modulating nitric oxide metabolism compositions comprising in addition to fluorocompounds modulating supplements including catalysts and/or renitrosation inhibitors (denitrosilases), nitrosation targets, reducers or combinations thereof are also claimed. The blood substitute "Perftoran" known before filing the previous application can be considered as a prototype for such compositions. The prototype drawback as regards the object of the given invention consists in an insufficient selectivity of modulating effect thereof i.e. impossible use thereof for varying (modulating) metabolism processes of nitric oxides toward desired direction without changing NO oxidation rate.

The proposal has an inventive step since earlier the effect of micellar catalysis for changing the system of equilibration of nitric oxides in NO oxidation was not known and $N_2O_3$ was considered as an acting nitrosating intermediate [24]. Catalytic and inhibiting effect of the claimed renitrosation modulators (e.g. methionine- and histidine-comprising peptides, polyphosphates and metallic complexes thereof) and their contribution into the system modulation of nitric oxides' equilibration was not known earlier. The composition in the form of emulsions the distribution curves by particle size of which have more than one maximum and/or prepared by mixing several compositions are also claimed. The advantage of such compositions consists in that each of the constituents can have an individual makeup and perform separate functions. For example, due to the fact that small micellae are phagocytized by some types of cells (e.g. macrophages) and large ones are not phagocytized by them, and alignment of component concentrations between individual micellae occurs rather slowly, metabolism of nitric oxides in both macrophage producing large amounts of NO and in environment thereof can be controlled independently using such composition.

A method for effecting organism of a patient in need of correcting metabolism of nitric oxides using the variants of the claimed method for modulating metabolism of nitric oxides and/or compositions for realizing thereof is concurrently claimed.

Numerous works are devoted to studying physiological effects of hydrophobic fluorocompounds especially emulsions based thereon that are used as blood substitutes. Mechanism of action thereof is usually explained by improved oxygen transport to tissues due to a better solubility thereof in hydrophobic phases than in water. The patent is also known [19] in which the use of perfluorocarbones' emulsions as nitric oxide depot or transporter is claimed. In the suggested invention the hydrophobic phases comprising one or more perfluorocompounds are the main medium for the course of NO oxidation reaction by oxygen. Due to micellar catalysis effect this reaction can be accelerated or slowed down as compared to the rate in absent perfluorocompounds. For this reason, higher nitric oxides are predominantly formed in these novel phases and just they are the sources of NO-equivalents possessing various physiologic effects. The claimed methods for effecting organism of a patient use the capabilities of nitric oxides' metabolism modulation method, i.e. of general or local modulating concentrations thereof in conformity with various cases. Since NO and nitrosothiols act as EDRF and participate in aggregation of platelets [5], the claimed methods can be used for preventing and treating ischemia, infarcts, strokes, blood coagulation pathology and hypertension. When preventing and treating atherosclerosis, one should take into consideration the fact that a lipid plaque on vascular wall also acts as a micellar NO oxidation catalyst and apoptosis modulator of surrounding cells [5]; therefore introduction of additional phases competing for a more efficient NO binding than that by tissue lipids is needed. Since theoretical upper limit for $Q_{NO}$ in lipids is less than 70 [2] and in perfluorocompounds it is higher than 70, then the use thereof is particularly efficient. Along with the combinations of the known medicinal effects (NO donors, inhibitors of NO-synthases) on the metabolism of nitric oxides the methods based on the effects of change in temperature and/or humidity are claimed. This effect mechanism is also connected with NO oxidation activation and NO metabolism changes elicited thereby. Under hyperthermic conditions organism protection against overheating consists in enhanced perspiration since cooling occurs in sweat evaporation. In simultaneous effect of a high humidity, however, evaporation is minimal that causes still a more active perspiration. Owing to the fact that the level of electrolytes in sweat is lower than in tissues, organism becomes dehydrated in sweating while concentration of solutes simultaneously rises (similar effect can be achieved by administering diuretics, distribution of solute concentrations in tissues, however, will be different). This results in enhanced efficacy of micellar catalysis in NO oxidation due to both increase in Q and increase in x (see Fig.1). At the same time rise in temperature leads to $N_2O_3$ dissociation and at higher temperatures dissociation of $N_2O_4$ also occurs that results in $NO_2$ concentration growth and activation of radical reactions that are an essential cytotoxicity factor (see Fig.2). This process is especially efficient in hydrophobic phases with high concentrations of perfluoroorganic compounds and with a high Q. Accordingly NO-inducible cytotoxicity can be increased using the suggested methods that can find application in treating infectious and cancer diseases. The options with a specific temperature regimen for a certain organ or a body surface part use the same principles. For example a tumor region can be heated for selective cytotoxicity enhancement or head can be cooled for lowering cytotoxicity and protecting cerebral cells.

Options with a specific atmospheric regimen are also based on controlling NO metabolism by micellar catalysis. In rising oxygen concentration the rates of both NO synthesis from arginine and NO oxidation increase and the reduction rate of nitrite and nitrosothiols falls [5]. Decrease in concentration acts in opposite direction. Quantitative concentration relations for these processes are different. In hydrophobic phases of fluorine comprising emulsions change in oxygen concentration controls only NO oxidation rate. $CO_2$ is a modulator of NO oxidation, nitration and nitrosation since it destabilizes peroxynitrite while bicarbonate is a $N_2O_3$ hydrolysis catalyst. $SF_6$ is a biochemically inert gas well soluble in hydrophobic phases and modulating solubility therein of other gases in particular $O_2$ and $CO_2$. Solubility of nitrogen in hydrophobic phases is different from solubility of $SF_6$ and thus metabolism of nitric oxides can be controlled by combination of partial pressures of these four gases. Air with NO admixture is used in clinical practice for ventilation and thus mixtures using all five gases ($O_2$, $N_2$, $CO_2$, $SF_6$ and NO) in combination with compositions of perfluorocompounds significantly expand capabilities of directed effect on metabolism of nitric oxides in various pathological conditions. Since lungs are one of the main organs of NO synthesis then a method using inhalation is claimed that can be used in treating bronchial asthma, hypoxia and pulmonary edema e.g. in mountain climbers.

Methods of effecting organism using the compositions for external application can be useful for treating burned patients and in surgical interventions, in inflammation when need in preventing cytotoxic effect of activated macrophages on damaged skin sites appears.

**Brief description of the invention drawings.**

Figure 1. Relation between increase of NO oxidation rate by oxygen in a heterogeneous system (H) and the portion of the hydrophobic phase (x) and the distribution coefficient (Q) between phases. Both increase (the left arrow) and decrease in total oxidation rate at all phases are possible resulting from changes in x and/or Q.

Figure 2. Main metabolism reactions of nitric oxides. NOS = NO-synthases, Hb = hemoglobin. Reaction 1 - NO biosynthesis by arginine oxidation, reactions 2-4 lead to irreversible removal of NO-equivalents from the cycle of nitric oxides, reaction 5 - the process activation of which is possible with micellar catalysis. Steady state NO concentrations and oxidation products depend on the presence of renitrosation catalysts and concentration of targets for both nitrosation and binding free radicals. Reduction rates of nitrosothiols (RSNO) depend on R, presence of catalysts, reducers and on oxygen concentration.

Figure 3. Typical kinetics of NO oxidation with oxygen in water and in a perfluorocarbonic compounds emulsion.

Figure 4. Change in nitrite and nitrate concentration in the course of experiment in the test group L-NAME.

Figure 5. Change in nitrite and nitrate concentration in the course of experiment in the control group L-NAME.

**Examples of realizing the invention**

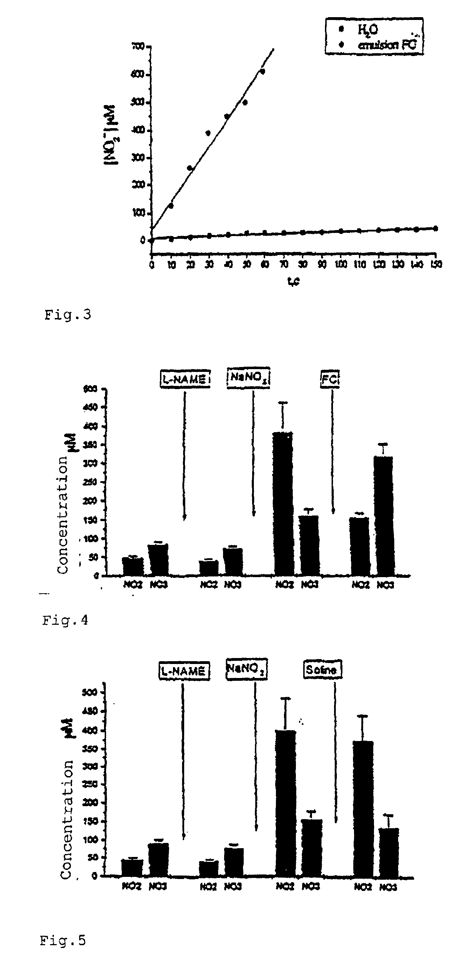

Kinetics of NO oxidation with oxygen in water, blood plasma, emulsions of perfluorocompounds in water and in blood plasma, following solubilization of perfluorohydrocarbones were obtained to illustrate the effect of micellar catalysis. Aqueous NO solution was added to reaction mixture kept in the air under atmospheric pressure. NO concentration was determined spectrophotometrically using hemoglobin and by nitrite quantity on completion of reaction. Fig.3 shows a typical kinetics of nitrite accumulation when sampling at 10-second interval. Nitrite and nitrate were determined in the products. Nitrite concentration was determined spectrophotometrically by azodye formation by Gris reaction at 540 nm immediately after sampling and blowing with argon. Nitrate was determined following reduction into nitrite. Examples 1-3 illustrate relation between initial NO oxidation reaction rates in the presence of emulsion of perfluorohydrocarbones and in distilled water ($W^0_{PFC}/W^0_{H2O}$). Examples 4 and 5 illustrate relation between initial NO oxidation reaction rates in the presence of perfluorohydrocarbones solubilized by blood plasma and in native plasma ($W^0_{PFC}/W^0_{plasma}$).

| Example | Heterogeneous mixture | $W^0_{PFC}/W^0_{H2O}$ |
|---|---|---|
| 1 | 1% perfluorodecaline emulsion | 63±15 |
| 2 | 1% perfluorooctylbromide emulsion | 45±10 |
| 3 | "Perftoran" - 20% emulsion with particle size 0.03-0.15 µm having the following makeup (in grams): perfluorodecaline, a mixture of isomers - 13; $C_{12}F_{23}N$ -perfluoromethyl cyclohexyl piperidine - 6.5; Proxanol-268 (Mw = 8 kD) - 4; NaCl - 0.6; $NaHCO_3$ - 0.065; KCl - 0.039; $MgCl_2$ - 0.019; $NaH_2PO_4$ - 0.02; glucose - 0.2; water - 100.0 | 40±10 |
| 4 | Perfluorodecaline solubilized in blood plasma | 25±5 |
| 5 | Perfluorooctyl bromide solubilized in blood plasma | 14±5 |

Example 6. In vivo micellar NO oxidation catalysis. (Because of indefinite concept "The best way of realizing an invention" example 6 if needed can be considered in the proposed group of inventions as such).

The experiment was performed on male rats of Wistar normotensive line weighing 180-400 grams. Polyethylene catheters were implanted to animals under Ketamine anesthesia (5mg/100g intraperitoneally) 24 hours prior to the experiment. The femoral artery was catheterized for connecting to arterial blood pressure (AP) transducer and the femoral vein was catheterized for intravenous administering medicinal preparations. AP and heart rate (HR) were measured in the abdominal aorta using a pressure transducer connected to a computer through an amplifier and a digital converter. The animals were divided into groups each consisting of a control and a test subgroups. The test subgroup was administered Perftoran (5 ml/kg), and the control group was administered similar quantity of a modified Krebs-Henseleit solution (KH). Monitoring AP and HR was continued for 1.5 hours after administering the solutions. Following recording initial AP level group one (group L-NAME) was intraperitonelly administered an inhibitor of NO-synthases $N^\omega$-nitro-L-arginine methyl ester (50 mg/kg) and in 1.5 hours when AP stabilized at a steady level $NaNO_2$ (1 mg/kg) was administered. After a steady lowering AP the animals were administered Perftoran or KH. Group two of rats (group FC) was administered Perftoran or KH immediately after recording initial AP level. Group three (group FCTh) was first administered 200 mg/kg α-lipoic acid and in 15 minutes it was administered Perftoran (or KH). Before commencement of each experiment, after AP got stabilized at a steady level while administering L-NAME and $NaNO_2$ and also immediately following completion of monitoring 0.2 ml blood were taken in each experiment from the arterial catheter for analysis.

**Group L-NAME.** In the both test (n=10) and control (n=9) subgroups mean arterial blood pressure (MAP) raised by 38±5 mm Hg following L-NAME administration. Administering $NaNO_2$ caused lowering MAP by 25±4 mm Hg in the both subgroups. Subsequent administering Perftoran the control subgroup caused a fast (within 10 minutes) rise in MAP up to the values characteristic of L-NAME effect and then a slow (for 30 to 40 minutes) significant lowering down to the initial level. At the same time administering KH solution the control subgroup against the background of L-NAME and $NaNO_2$ effect resulted in a gradual insignificant rise in MAP. Plasma levels of nitrites and nitrates showed insignificant lowering after NOS inhibitor administration; following $NaNO_2$ injection nitrite level increased by 344±68 µM in experimental animals and by 362±73 µM in the controls; that of nitrate increased by 87±15 µM in experimental animals and by 76±26 µM in the controls. Administering the experimental subgroup Perftoran resulted in a significant change in nitrite-nitrate blood balance: nitrite level dropped by 220±40 µM and nitrate level raised by 157±12 µM. Administering the control group KH solution resulted in insignificant (33±13 µM) lowering plasma nitrite and nitrate levels (see Figures 4 and 5).

**FC Group.** Administering the control subgroup Perftoran also resulted in change in blood nitrite/nitrate balance. Against the background, nitrite level rise by 49±6 µM nitrate concentration lowered by 23±8 µM. As a result the total level of NO oxidation raised. Administering the control subgroup KH resulted in a homogenous insignificant lowering concentration of the both ions by 7±4 µM.

Thus in the both groups of experiments administering a new phase with a higher value of distribution coefficients Q into blood resulted in accelerated NO oxidation. In NAME group the own NO synthesis as affected by NO-synthases was inhibited and NO could be formed only in reducing nitrite and thionitrites (see Fig.2). Activation of NO oxidation with micellar catalysis in hydrophobic phases resulted in the growth of $NO_2$ concentrations and therefore, nitrite concentration reduced and nitrate concentration raised. In the absence of NO-synthases' inhibitor biogenic NO shifted the balance toward $N_2O_3$ while nitrite concentration growing and nitrate concentration dropping.

**FCTh group.** α-lipoic acid induced insignificant lowering MAP in the both subgroups, however, Perftoran administered to the test subgroup (n=10) against the background of thiol effect resulted in a fast rise in MAP by 16±4 mm Hg and then in a gradual significant drop in MAP by 8±3 mm Hg below the initial level. In the control subgroup

**EP 1 214 933 A1**

(n=10) an insignificant rise in MAP was observed up to the initial level against the background of KH administration. The same MAP values were also maintained in the control in 1.5 hours after the solution administration. Administering Perftoran significantly rises nitrite concentration (46±6 μM), but not that of nitrate. Thus rise in the total blood nitrite and nitrate levels is still more significant than in FC group.

Example 6 shows the possibilities of practical realizing the invention in order to accelerate NO oxidation in blood when administering perfluorocompounds and the effect of modifying supplements of NO-synthases and targets for nitrosation.

The efficacy of renitrosation catalysts was determined by $N^1$-nitrozotriptophan (NOW) denitrosation kinetics in the composition of an acylic derivative or serum albumin at 32°C and at different pH values by absorption decrease at $\lambda$ = 340 nm or by differential spectroscopy relative to denitrosation reaction in a buffer solution, in relation to nitrite/nitrate ratio or by the ratio analysis of nitrosation products. Examples 7-17 illustrate catalytic and inhibitory activity of $W^0/W^0_{buffer}$ compounds used in the claimed compositions (normalized for the buffer molarity).

| Example | Substance | Reference buffer, pH | $W^0/W^0_{buffer}$ |
|---|---|---|---|
| 7 | $Na_5P_3O_{10}$ | Phosphate, 6.3 | 4 |
| 8 | $Na_2MnATP$ | Phosphate, 6.3 | 0.7 |
| 9 | $Na_3MnP_3O_{10}$ | Phosphate, 6.3 | 21 |
| 10 | $Na_3MgP_3O_{10}$ | Phosphate, 6.3 | 11 |
| 11 | Ala-Met | MOPS, 6 MOPS, 6 | 18 |
| 13 | D-Arg | MOPS, 6 MOPS, 6 | 0.56 |
| 14 | Cly-Asp | MOPS, 6 | 15 |
| 15 | Gly-His | Hepes, 6.1 | 16 |
| 16 | Met-Glu-His-Phe-Pro-Gly-Pro | Phosphate, 5.9 | 310 |
| 17 | Ascorbic acid | Phosphate, 5.9 | 32 |

Examples 18-34 illustrate various compositions prepared by modifying the basic compositions. Crystallization water is not indicated; all the amino acids are of L-series.

| Example | Initial composition | Added (+) or removed (-), in mg per 100 g | Notes |
|---|---|---|---|
| 18 | Perftoran (Example 3) | + 1200 $SF_6$ | Administered in the form of gas |
| 19 | | + 1000 perfluorooctylbromide + 400 phospholipid | Administered in the form of emulsion with aqueous phase of Perftoran, average micellar size 50 μm |
| 20 | | +100 fructose +30 ascorbic acid +30 sodium ascorbate +100 arginine +50 lipoic acid | |
| 21 | | +50 Gly-Asp +50 Gly-His +50 Ala-Met +300 ascorbic acid | |
| 22 | | +30 $Na_5P_3O_{10}$ +200 $Na_2MnATP$ +200 Gly-Gly-$NH_2$ | |
| 23 | | +30 $Na_2AMP$ +50 $Na_{2Mg}ATP$ | |
| 24 | | +100 Met-Glu-His-Phe-Pro-Gly-Pro | |
| 25 | | +2300 ascorbic acid +1000 sodium ascorbate | |

(continued)

| Example | Initial composition | Added (+) or removed (-), in mg per 100 g | Notes |
|---|---|---|---|
| 26 | | +1000 perfluorodecaline +1000 ascorbic acid +50 tocopherol acetate +50 dithiotreitol +400 phospholipid +5 retinol acetate +120 trioleine +500 sodium sulfamate +500 Met-Glu-His-Phe-Pro-Gly-Pro | |
| 27 | 26 | +200 $Na_3MNP_3O_{10}$ +100 thiourea +50 sodium carbamate +50 sodium amidophosphate | |
| 28 | 25 | +50 Ala-Met +500 Met-Ala +His-Gly +50 Asn +50 Asp +50 Gln +50 Glu-Na +20 dihydrolipoic acid | |
| 29 | | +200 pentadecylamine +50 diamine butane +20 dithiopropanol +1000 ascorbic acid | |
| 30 | | - glucose +200 fructose +500 ascorbic acid +50 tocopherol acetate | |
| 31 | | +500 serum albumin | |
| 32 | Perfluorode caline | +5000 $SF_6$ +50 thiourea +50 dihydrolipoic acid +350 tocopherol | Suspended by ultrasound, $SF_6$ administered in the form of gas |
| 33 | Mixture of equal portions of the compositions 24 and 25 | | |
| 34 | Mixture of equal portions of the compositions 18-32 | | |

Examples 35-38 illustrate a method for effecting organism of a patient.

Example 35. Compresses comprising the composition 20 and a placebo and comprising no perfluorocompounds were applied on inflamed scratches (n=6) of similar area. In 24 hours the scratches with applied composition 20 did not show inflammation signs as compared to the controls.

Example 36. A classic log Russian steam bath was used the bath being equipped with a hearth having a 100 liter capacity boiler located over the hearth. Stones fastened together by clay solution surround the hearth and the boiler. The bath is "black" stoked with birch firewood and ventilated for 1.5 hours after stocking. Average temperature on the bath shelf was 65°C at 70% humidity; patients were exposed to 2 runs 12 minutes each in a stationary regimen with a 10-minute inter-run interval at 37°C and 88% humidity. 150 µl 0.1% aqueous solution of a peptide renitrosation catalysts comprising 80% Met-Glu-His-Phe-Pro-Gly-Pro and 20% Met (S=O) -Glu-His-Phe-Pro-Gly-Pro (the mixture of steroisomers by sulphur atoms was intranasally administered into each nostril of the test group patients (n=4) one hour before the run and immediately after the first run; the control group (n=4) was administered placebo. One hour after completion of the second run the rate of response and capability of mobilizing attention were checked in a series of control tests with tying an asymmetric knot. The rate of correct fulfillment of the test in the test group was 2.4-fold higher than in the control group.

Example 37. The classic bath according to example 36 was used, 2 runs 12 minutes each as in example 36 were performed. A sum of concentrations (nitrite + nitrate) was determined in urine samples collected immediately before run one and 30 minutes after completion of run two as a measure of NO oxidation intensity. In sample two the sum was 1.3-fold higher.

Example 38. The bath according to example 36 was used, 3 runs 12 minutes each as in example 36 were performed. 0.5 ml the composition according to example 33 were additionally administered to the test group by inhalation and head cooling by applying on top a sealed polyethylene bag loosely filled with ice. The experiment participant himself controlled cooling intensity by achieving a maximum comfortable condition. In the asymmetric knot tying test the rate of correct test performing in the test group was 4.3-fold higher than in the control group.

## EP 1 214 933 A1

**Industrial applicability.**

Implementation of the suggested inventions is practically possible. Perfluorocarbons are allowed for using as blood substitutes while amounts of perfluorocarbons being administered into blood circulation are significantly larger than is required for achieving the maximum reaction acceleration (the maximum H value) in accordance with the equation for micellar catalysis efficacy. Unite dosage forms based on the stabilized emulsions of fluorinated organic compounds are commercially produced and approved for clinical application [18]. The claimed compositions are practically applicable since inert biologically compatible fluorine comprising compounds and used as hydrophobic phases as well as stabilizers of emulsions are produced by industry; methods for obtaining highly disperse emulsions based on them are also known. Every class of modifying supplements included in the compositions has representatives among the biologically compatible and non-toxic natural or synthetic compounds produced by industry. Fragments of natural human hormones or synthetic analogs thereof, e.g. nootropic drug "Semax" (Met-Glu-His-Phe-Pro-Gly-Pro), a peptide comprising methyonyl-histidyl, belong to the compounds possessing denitrosilase activity. Reducers (for example ascorbic acid) and scavengers of free radical (for example tocopherol acetate) produced by industry are available. Arsenal of commercially produced targets for nitrosation including those used as pharmaceutical preparations, is also broad (for example lipoic and dihydrolipoic acids). Artificial hyper- or hypothermia is widely used both clinically and in folk medicine (for example in steam bath and sauna).

**Bibliography**

[I] Liu, X., et al (1998) *Proc. Natl. Acad.* Sci. USA **95,**2175-2179.

[2] Gordin, V.A., Nedospasov, AA. (1998) *FEBS Letters* **424,**239-242.

[3] Gow, AJ., Stamler, J.S. (1998) *Nature* **391,**169-173.

[4] Mayer, B., Hemmens;B.N. (1997) *Trends Biochem. Sci.* **22,**477-481.

[5] Nedospasov, A.A. (1998) *Biochemistry (Moscow)* **63,**881-904.

[6] Kuhl, S J., Rosen, H. (1998) *West. J. Med.* **168,**176-181.

[7] Pryor, W.A., Squadrito, GX. (1995) *Am. J. Physiol.* **268,** L699-L722.

[8] Xie, K., Fidler, IJ. (1998) *Cancer Metastasis Rev* **17**,55-75.

[9] Gorbunov, N.V. et al (1998) *Biochem. Biophys. Res. Commun.* **244**,647-651.

[10] Darley-Usmar, V., Wiseman, H., Halliwell, B. (1995) *FEBS Letters* **369**,131-135.

[II] Stuehr, DJ. (1997) *Annu. Rev. Pharmacol.* Toxicol. **37,**339-359.

[12] Liu, X., et al. (1998) *J. Biol. Chem.* **273,**18709-18713.

[13] Reutov, V.P., Sorokina, E.G., Kaiushin, L.P. (1994) *Vop. Med Khim.* **40,**31-35.

[14] Singh, R.J., Hogg, N., Joseph, J., Kalyanaraman, B. (1996) *J. Biol. Chem.* **271,** 18596-18603.

[15] Kashiba-Iwatsuki, M, et al (1997) *J. Biochem. (Tokyo)* **122,** 1208-1214.

[16] Whittle, BJ. (1995) *Histochem. J.* **27,**727-737.

[17] Beda, N.V., Suntsova, T.P. (1999) *FEBS Letters* **453,**229-235.

[18] Patent RU 2088217 Cl, 27.08.1997

[19] Patent USA 5,726,209 (1998)

[20] Adv. in blood substitute research, (eds Bolin RJB. et al) Alan R.Liss, Inc., NY(1983).

[21] Riess, J.G. (1994) *Artif. Cells Blood Substit Immobil. Biotechnol.* **22,**215-34.

[22] Patent USA. 5,733,939 (1998).

[23] Patent USSR 939440.

[24] Caulfield, J.L., et al (1996) *J. Biol. Chem.* **271,**25859-25863.

**Claims**

1. A method for modulating metabolism of nitric oxides by varying NO oxidation rate in a heterogeneous medium by changing makeup thereof **characterized in that** the number of phases in this medium and/or one or more volume ratios of phases and/or one or more NO or oxygen distribution coefficients between phases are modified.

2. The method according to claim 1 **characterized in that** for accelerating NO oxidation with oxygen the changes are carried out in such a way that the values of the expression

$$H = \frac{\sum_{i=1}^{i=n-1} k_i Q_{NO,i}^2 Q_{O_2,i} x_i + k_n \left(1 - \sum_{i=1}^{i=n-1} x_i\right)}{\left(1 + \sum_{i=1}^{i=n-1} Q_{NO,i} x_i - \sum_{i=1}^{i=n-1} x_i\right)^2 \left(1 + \sum_{i=1}^{i=n-1} Q_{O_2,i} x_i - \sum_{i=1}^{i=n-1} x_i\right)}$$

*where H is acceleration of NO oxidation reaction with oxygen in heterogeneous n-phasic system as compared to the least hydrophobic (aqueous) phase, $k_i$ is reaction rate constant in i phase, $Q_{Noi}$, $Q_{o2i}$ is a equilibrated distribution coefficient of NO and $O_2$ in i phase, $x_i$ is a portion of i phase in a total volume,*
would increase and for slowing down NO oxidation with oxygen the changes are carried out in such a way that the H value would decrease.

3. The method according to any one of claims 1 and 2 **characterized in that** for changing NO distribution coefficients between the phases the medium quantitative makeup is changed without changing qualitative makeup and/or without forming novel phases.

4. The method according to any one of claims 1 to 3 **characterized in that** one or more components are introduced into heterogeneous medium so that one or more novel phases appear therein.

5. The method according to any one of claims 1 to 4 **characterized in that** the components being introduced comprise one or more compounds selected from the group consisting of: a perfluorohydrocarbone, a halo-substituted perfluorocarbohydra-te derivative and a tertiary perfluoroalkylamine, $SF_6$.

6. The method according to any one of claims 1 to 5 **characterized in that** the components being introduced comprise solution of a protein that solubilizes the fluorinated organic compound having the value of distribution coefficients $Q_{NO}$ and/or $Q_{O2}$ in a biphasic system with water higher than the maximum value of $Q_{NO}$ and/or $Q_{O2}$ for arbitrary pair of phases of reaction mixture before introducing.

7. The method according to any one of claims 1 to 6 **characterized in that** the heterogeneous medium is blood plasma.

8. The method according to any one of claims 1 to 6 **characterized in that** the heterogeneous medium is blood.

9. The method according to any one of claims 1 to 8 **characterized in that** the components comprising one or more catalysts and/or renitrosation inhibitors are additionally introduced.

10. The method according to any one of claims 1 to 9 **characterized in that** the components comprising one or more reducers are additionally introduced.

11. The method according to any one of claims 1 to 10 **characterized in that** the components comprising one or more scavengers of free radicals are additionally introduced.

12. The method according to any one of claims 1 to 11 **characterized in that** the components comprising one or more compounds producing a nitrosocompound and/or releasing nitrogen as affected by NO oxidation products are additionally introduced.

13. The method according to any one of claims 1 to 12 **characterized in that** additionally the temperature of the heterogeneous medium or of a portion thereof is modified.

14. Compositions comprising a perfluororganic compound resistant in metabolic reactions and forming with water a heterogeneous mixture said compound being selected from the group including: perfluorohydrocarbons, halo-derivatives of perfluorohydrocarbons, 0.1 to 90% perfluoroalkylamines and one or more compounds of the group: SF$_6$, perfluorohydrocarbons, halo-derivatives of perfluorohydrocarbons, tertiary perfluoroalkylamines, water up to 100% for modulating metabolism of nitric oxides.

15. The composition according to claim 14 **characterized in that** one or more compounds belonging to one or more groups from the following list are additionally introduced therein: emulsifiers, biologically compatible salts for maintaining pH and/or ionic strength, carbohydrates for maintaining osmotic pressure and/or one or more compounds belonging to one or more groups of the following list: catalysts or inhibitors of pernitrosifiation, reducers, scavengers of free radicals, targets for nitrosation and/or precursors thereof, targets for nitrosation with nitrogen release.

16. The composition according to claim 15 **characterized in that** copolymers of ethylene oxide and of propylene oxide and/or phospholipids are introduced as emulsifiers.

17. The compositions according to any one of claims 15 and 16 **characterized in that** they are emulsions of the fluorine comprising compounds with average size of micellae less than 100 nm.

18. The compositions according to any one of claims 15 to 17 **characterized in that** glucose and/or fructose and/or saccharose are introduced as carbohydrates for maintaining osmotic pressure.

19. The compositions according to any one of claims 15 to 18 **characterized in that** ascorbic acid and/or salts thereof and/or retinol and/or acylic derivatives thereof are introduced as reducers.

20. The compositions according to any one of claims 15 to 19 **characterized in that** one or more substituted or unsubstituted mono- and/or di- and/or polyphosphates and/or complexes thereof with magnesium or zinc or copper or manganese are introduced as catalysts or renitrosation inhibitors.

21. The compositions according to any one of claims 15 to 20 **characterized in that** one or more compounds of the group: thiourea, thioamides, methionine, arginine, peptides and/or acylic and/or amide dervatives thereof of general formula X-Pept-Y, where X=H or acyl, Y=OH or -NH$_2$ or NHR or NR$_1$R$_2$, Pept=peptide comprising residues of methionine and/or aspartic acid and/or histidine and/or glutamic acid and/or arginine, are introduced as catalysts or renitrosation inhibitors.

22. The composition according to claim 21, **characterized in that** Pept comprises a fragment Met-Glu-His-Phe.

23. The composition according to any one of claims 21 or 22, **characterized in that** Pept is Met-Glu-His-Phe-Pro-Gly-Pro and all the amino acids belong to L-series.

24. The composition according to any one of claims 15-23 **characterized in that** tocopherol and/or acylic derivatives thereof are introduced as scavengers of free radicals.

25. The composition according to any one of claims 15-24 **characterized in that** one or more thiols or dithiols or disulphides are introduced as targets for nitrosation and/or precursors thereof.

26. The composition according to any one of claims 15-25 **characterized in that** one or more compounds from the group: dithiopropanol, dithiobutanediol, lipoic acid, dihydrolipoic acid, cysteine, homocysteine, peptides comprising cysteine and/or cystine, acylic and/or esteric and/or amide derivatives of cysteine or cystine or peptides comprising these amino acids or protein, are introduced as targets for nitrosation and/or precursors thereof.

27. The compositions according to any one of claims 15-26 **characterized in that** one or more compounds from the group: urea, sulfaminic acid and salts thereof, amidophosphoric acid and salts thereof, carbamic acid and salts thereof, asparagine, aspartic acid and salts thereof, glutamine and salts thereof, glutaminic acid and salts thereof, peptides comprising asparagine and/or glutamine, primary amine or salts thereof are introduced as targets for nitrosation with nitrogen release.

28. The compositions according to any one of claims 15-26 **characterized in that** they are emulsions that have distribution dependencies of the number of particle by size with more than one maximum and/or they are compositions prepared by mixing two or more compositions according to claims 14-27.

**29.** A use of blood substitutes based on stabilized emulsions of the fluorine comprising compounds for modifying NO oxidation rate.

**30.** A method for effecting organism of a patient in need of correcting metabolism of nitric oxides **characterized in that** for modifying NO oxidation rates and subsequent reaction the number of phases is modified in the organism and environment thereof and/or one or more ratios between volumes of the phases and/or one or more distribution coefficients of NO or oxygen between the phases.

**31.** The method according to claim 30 **characterized in that** a patient is orally administered a composition comprising one or more water immiscible fluorine comprising compounds and resistant in metabolism reactions and/or this composition is used locally for contacting a skin tegument site or a wound.

**32.** The method according to claim 31 **characterized in that** one or more compounds from the group: water, emulsifier, biologically compatible salts maintaining pH and/or ionic strength, carbohydrates for maintaining osmotic pressure and/or one or more compounds belonging to one or more groups of the following list: catalysts or inhibitors of pernitrosifiation, reducers, scavengers of free radicals, targets for nitrosation and/or precursors thereof, targets for nitrosation with nitrogen release.

**33.** The method according to any one of claims 31 and 32 **characterized in that**

one or more compounds from the group:a perfluorohydrocarbon, a perfluorohydrocarbon halo-substituted derivative and a tertiary perfluoroalkylamine are introduced as the fluorine comprising water immiscible compounds;
copolymers of ethylene oxide and of propylene oxide and/or phospholipids are introduced as emulsifiers;
and/or glucose and/or fructose and/or saccharose are introduced as carbohydrates for maintaining osmotic pressure;
and/or ascorbic acid and/or salts thereof and/or retinol and/or acylic derivatives thereof are introduced as reducers;
and/or one or more substituted or unsubstituted mono- and/or di - and/or polyphosphates and/or complexes thereof with magnesium or zinc or copper or manganese are introduced as catalysts or renitrosation inhibitors;
and/or thiourea, thioamides, methionine, arginine, peptides and/or acylic and/or amide derivatives thereof of general formula X-Pept-Y, where X=H or acyl, Y=OH or -$NH_2$ or NHR or $NR_1R_2$, Pept=peptide comprising residues of methionine and/or aspargic acid and/or histidine and/or glutamic acid and/or arginine, and/or Pept comprises a fragment Met-Glu-His-Phe; and/or Pept = Met-Glu-His-Phe-Pro-Gly-Pro are introduced as catalystst or renitrosation inhibitors;
and/or tocopherol and/or acylic derivatives thereof are introduced as scavengers of free radicals;
and/or one or more thiols or dithiols or disulphides and/or one or more compounds from the group: dithiopropanol, dithiobutanol, lipoic acid, dihydrolipoic acid, cysteine, homocysteine, peptides comprising cysteine or cystine, acylic and/or esteric and/or amide derivatives of cysteine or cystine or peptides comprising these amine acids, or protein are introduced as the targets for nitrosation and/or precursors thereof;
and/or one or more compounds from the group: urea, glutamic, aspartic, carbamic, amidophosphoric, sulfamic acids and salts thereof, asparagine, glutamine, primary amine and salts thereof, peptides comprising asparagine and/or glutamine are introduced as the targets for nitrosation with nitrogen release.

**34.** The method according to any one of claims 31-33 **characterized in that** several compositions are used simultaneously or sequentially and/or the compositions are emulsions that have distribution dependencies of the number of particle by size with more than one maximum and/or they are compositions prepared by mixing two or more compositions according to any one of claims 30-32.

**35.** The method according to any one of claims 31-34 **characterized in that** the composition is administered intravenously.

**36.** The method according to any one of claims 31-35 **characterized in that** the composition is administered by inhalation.

**37.** The method according to any one of claims 30-36 **characterized in that** a patient is additionally administistered catalysts or renitrosation inhibitors.

**38.** The method according to any one of claims 30-37 **characterized in that** a patient is additionally administered diuretics.

**39.** The method according to any one of claims 30-38 **characterized in that** a patient is additionally administered inhibitors of NO-synthases.

**40.** The method according to any one of claims 30-39 **characterized in that** a patient is additionally administered NO donors and/or NO inhalation is performed.

**41.** The method according to any one of claims 30-40 **characterized in that** a patient or one or more individual organs and/or body parts are additionally subjected to the effect of hypothermia and/or infrared irradiation.

**42.** The method according to any one of claims 30-41 **characterized in that** a patient or a part of his body surface are under the conditions of increased relative humidity.

**43.** The method according to any one of claims 30-42 **characterized in that** a patient or one or more individual organs and/or body parts are additionally subjected to the effect of low temperature.

**44.** The method according to any one of claims 30-43 **characterized in that** a patient and/or a part of body surface and/or an individual organ are additionally subjected to the effect of a gas mixture of oxygen with one or more gases from the group of $SF_6$, $N_2$, $CO_2$ NO at partial pressures thereof different from the usual ones in the air makeup at atmospheric pressure and/or said gas mixture is used for respiration of the patient.

**45.** The method according to any one of claims 30-44 **characterized in that** the patient belongs to the group at risk of septic shock or under the condition of septic shock.

**46.** The method according to any one of claims 30-44 **characterized in that** the patient belongs to the group at risk of hypoxia or pulmonary edema.

**47.** The method according to any one of claims 30-44 **characterized in that** the patient belongs to the group at risk of cancer disease or cancer patients.

**48.** The method according to any one of claims 30-44 **characterized in that** the patient belongs to the group of patients suffering from bronchial asthma.

**49.** The method according to any one of claims 30-44 **characterized in that** the patient belongs to the group of patients suffering from infectious diseases.

**50.** The method according to any one of claims 30-44 **characterized in that** the patient belongs to the group of burned patients.

**51.** The method according to any one of claims 30-44 **characterized in that** the patient belongs to the group of patients in postoperative period.

**52.** The method according to any one of claims 30-44 **characterized in that** the patient belongs to the group at risk of stroke or of those who sustained stroke.

**53.** The method according to any one of claims 30-44 **characterized in that** the patient belongs to the group at risk of infarction or of those who sustained infarction.

**54.** The method according to any one of claims 30-44 **characterized in that** the patient belongs to the group at risk of atherosclerosis or patients suffering from atherosclerosis.

**55.** The method according to any one of claims 30-44 **characterized in that** the patient belongs to the group at risk of blood coagulation pathology or to patients suffering from blood coagulation pathology.

**56.** A use of steam bath or sauna for modulating metabolism of nitric oxides by modifying NO oxidation rate.

Fig.I

Fig.2

Fig.3

Fig.4

Fig.5

## INTERNATIONAL SEARCH REPORT

| | International application No |
|---|---|
| | PCT/RU 00/00362 |

**A. CLASSIFICATION OF SUBJECT MATTER :**
IPC 7    A61K9/107, A61K31/02

According to International Patent Classification (IPC) or to both national classification and IPC

**B. FIELDS SEARCHED**

Minimum documentation searched (classification system followed by classification symbols)
IPC 7    A61K31/00, 31/02, 31/13, 33/16, 35/14, 35/16, 9/00, 9/107, 9/113, 31/375

Documentation searched other than minimum documentation to the extent that such documents are included in the fields searched

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 96/30012 A1 (DEFEUDIS, FRANCIS, V.)   3 October 1996 (03.10.96), the claims | 1-13, 30-55 |
| A | EP 0307087 A1 (LONG, DAVID V., JR.)  15 March 1989 (15.03.89), the claims | 14-28 |
| A | WO 97/38579 A1 (BOARD OF REGENTS et al)  23 October 1997 (23.10.97), the claims | 14-28, 29 |
| A | WO 96/40058 A3 (ALLIANCE PHARMACEUTICAL CORP.) 19 December 1996 (19.12.96), the abstract | 29 |
| A | Malaya meditsinskaya entsiklopedya, under editorship V.I. POKROVSKOGO, volume 1, M. Sovetskaya entisiklopedya, 1991, pages 206-207 | 56 |

☐ Further documents are listed in the continuation of Box C     ☐ See patent family annex.

* Special categories of cited documents:

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search report | Date of mailing of the international search report |
|---|---|
| 25 December 2000 (25.12.00) | 11 Januar 2001 (11.01.01) |

| Name and mailing address of the ISA/ | Authorized officer |
|---|---|
| R U | |
| Facsimile No. | Telephone No. |

Form PCT/ISA/210 (second sheet) (July 1992)